# EUROPEAN PATENT APPLICATION

(11) **EP 4 837 566 A2**
(43) Date of publication of application: **11.11.2026**
(21) Application number: 26196331.8
(22) Date of filing: 08.11.2017
(51) Int. Cl.: A61K 9/20

(54) **PHARMACEUTICAL COMPOSITION, METHODS FOR TREATING AND USES THEREOF**

(30) Priority: 10.11.2016 US 201662420062 P; 02.06.2017 US 201762514249 P
(62) Document number(s) of the earlier application(s) in accordance with Art. 76 EPC: 17797137.1 / 3 538 107
(71) Applicant: Boehringer Ingelheim International GmbH, 55216 Ingelheim am Rhein (DE)
(72) Inventor: VON EYNATTEN, Maximilian, 55216 INGELHEIM AM RHEIN (DE); BROEDL, Uli Christian, 55216 INGELHEIM AM RHEIN (DE); WOERLE, Hans-Juergen, 55216 INGELHEIM AM RHEIN (DE)
(74) Representative: Boehringer Global Patents

(57) **Abstract**

The present invention relates to methods for treating or preventing chronic kidney disease and cardiovascular disease in patients with chronic kidney disease comprising administering empagliflozin to the patient.

## Description

### Technical Field of the Invention

The present invention relates to methods for treating or preventing chronic kidney disease and cardiovascular disease in patients with chronic kidney disease.

### Background of the Invention

Chronic kidney disease (CKD), also known as chronic renal disease, is a progressive loss in renal function over a period of months or years. The symptoms of worsening kidney function are non-specific, and chronic kidney disease is often diagnosed as a result of screening of people known to be at risk of kidney problems. CKD is a highly prevalent disease, afflicting more than one out of ten individuals worldwide.

Chronic kidney disease may be identified by a blood test, for example for creatinine. Higher levels of creatinine indicate a lower glomerular filtration rate and as a result a decreased capability of the kidneys to excrete waste products.

CKD has been classified into 5 stages, where stage 1 is kidney damage with normal GFR (mL/min/1.73 m²) of ≥ 90; stage 2 is kidney damage with a mild decrease in GFR (GFR 60-89); stage 3 is a moderate decrease in GFR (GFR 30-59); stage 4 is a severe decrease in GFR (GFR 15-29); and stage 5 is kidney failure (GFR <15 or dialysis). Stage 5 CKD is often called End Stage Renal Disease (ESRD) and is synonymous with the now outdated terms chronic kidney failure (CKF) or chronic renal failure (CRF).

Albuminuria can also be a sign of kidney disease. Albuminuria has been classified into 3 categories, where category A1 reflects no albuminuria with albumin normal to mildly increased; category A2 which reflects microalbuminuria with albumin moderately increased; category A3 which reflects macroalbuminuria with albumin severely increased.

There is no specific treatment unequivocally shown to slow the worsening of chronic kidney disease and severe CKD requires renal replacement therapy, which may involve a form of dialysis, but ideally constitutes a kidney transplant.

Since many CKD patients will die due to a cardiovascular event (CV) event before reaching ESRD, reducing CV risk is another consideration in treatment.

Therefore, there is an unmet medical need for methods, medicaments and pharmaceutical compositions able to slow the worsening or progression of chronic kidney disease and reduce the risk of CV events in patients, in particular patients with chronic kidney disease.

### Summary of the Invention

The present invention provides a method of treating or slowing the progression of chronic kidney disease in a patient with chronic kidney disease, the method comprising administering empagliflozin to the patient. In one aspect, the method additionally reduces the risk of cardiovascular death in the patient. In one aspect, the method additionally reduces the risk of all-cause mortality in the patient. In one aspect, the method additionally reduces the risk of all-cause hospitalization in the patient. In a further aspect, the patient has moderately or severely decreased renal function or elevated albuminuria levels, for example ≥200 mg/g.

In one aspect, the present invention also provides a method for reducing the risk of chronic kidney disease, the method comprising administering empagliflozin to the patient. In one aspect, the method additionally reduces the risk of cardiovascular death in the patient. In one aspect, the method additionally reduces the risk of all-cause mortality in the patient. In one aspect, the method additionally reduces the risk of all-cause hospitalization in the patient. In a further aspect, the patient has moderately or severely decreased renal function or elevated albuminuria levels, for example ≥200 mg/g.

In one aspect, the patient has an eGFR ≥20 to <45 mL/min/1.73 m². In one aspect, the patient has an eGFR ≥20 mL/min/1.73 m² and a urinary albumin-to-creatine ratio (UACR) ≥200 mg/g. In one aspect, the patient has an eGFR ≥45 and <90 ml/min/1.73m² and a urinary albumin-to-creatine ratio (UACR) ≥ 200mg/g.

In one aspect, the patient is treated with a RAAS inhibitor (Renin-Angiotensin-Aldosterone System). In one aspect, the patient is treated with an Angiotensin-Converting Enzyme (ACE) inhibitor and/or an angiotensin II receptor blocker (ARB).

In one aspect, the patient is on standard of care according to local/international guideline to treat CKD risk factors.

In one aspect, the patient is a non-diabetic patient. In one aspect, the patient is a patient with pre-diabetes or a patient with type 2 or type 1 diabetes mellitus.

In one aspect, the patient is a non-diabetic and non-pre-diabetic patient. In one aspect, the patient is not at risk or even high risk for cardiovascular events. In one aspect, the patient is a not a patient with chronic heart failure, in particular not a patient with HFrEF (Heart Failure with reduced Ejection Fraction) and/or HFpEF (Heart Failure with preserved Ejection
Fraction).

In one aspect, empagliflozin is administered at a dose in a range from 0.5 mg to 25 mg, for example from 1 mg to 25 mg, for example at a dose of 10 mg or 25 mg. In one aspect, empagliflozin is administered once daily to the patient.

The present invention further provides for empagliflozin or a pharmaceutical composition comprising empagliflozin optionally in combination with one or more other therapeutic substances for use as a medicament in any one of the methods described herein.

The present invention further provides for empagliflozin or a pharmaceutical composition comprising empagliflozin optionally in combination with one or more other therapeutic substances for use in a method for treatment, prevention, slowing the progression or risk reduction in any one of the diseases or conditions described herein.

The present invention further provides for empagliflozin or a pharmaceutical composition comprising empagliflozin optionally in combination with one or more other therapeutic substances for use in the manufacture of a medicament for use in any one of the methods described herein.

In the methods according to the present invention empagliflozin is optionally administered in combination with one or more other therapeutic substances to the patient.

Further aspects of the present invention become apparent to the one skilled in the art by the description hereinbefore and in the following and by the examples.

### Definitions

The term **"active ingredient"** of a pharmaceutical composition according to the present invention means the SGLT2 inhibitor empagliflozin according to the present invention. An "active ingredient" is also sometimes referred to herein as an "active substance".

The term **"body mass index" or "BMI"** of a human patient is defined as the weight in kilograms divided by the square of the height in meters, such that BMI has units of kg/m².

The term **"overweight"** is defined as the condition wherein the individual has a BMI greater than or 25 kg/m² and less than 30 kg/m². The terms "overweight" and "pre-obese" are used interchangeably.

The terms **"obesity"** or **"being obese"** and the like are defined as the condition wherein the individual has a BMI equal to or greater than 30 kg/m². According to a WHO definition the term obesity may be categorized as follows: the term "class I obesity" is the condition wherein the BMI is equal to or greater than 30 kg/m² but lower than 35 kg/m²; the term "class II obesity" is the condition wherein the BMI is equal to or greater than 35 kg/m² but lower than 40 kg/m²; the term "class III obesity" is the condition wherein the BMI is equal to or greater than 40 kg/m².

The indication obesity includes in particular exogenic obesity, hyperinsulinaemic obesity, hyperplasmic obesity, hyperphyseal adiposity, hypoplasmic obesity, hypothyroid obesity, hypothalamic obesity, symptomatic obesity, infantile obesity, upper body obesity, alimentary obesity, hypogonadal obesity, central obesity, visceral obesity, abdominal obesity.

The term **"visceral obesity"** is defined as the condition wherein a waist-to-hip ratio of greater than or equal to 1.0 in men and 0.8 in women is measured. It defines the risk for insulin resistance and the development of pre-diabetes.

The term **"abdominal obesity"** is usually defined as the condition wherein the waist circumference is > 40 inches or 102 cm in men, and is > 35 inches or 94 cm in women. With regard to a Japanese ethnicity or Japanese patients abdominal obesity may be defined as waist circumference ≥ 85 cm in men and ≥ 90 cm in women (see e.g. investigating committee for the diagnosis of metabolic syndrome in Japan).

The term "euglycemia" is defined as the condition in which a subject has a fasting blood glucose concentration within the normal range, greater than 70 mg/dL (3.89
mmol/L) and less than 100 mg/dL (5.6 mmol/L). The word "fasting" has the usual meaning as a medical term.

The term "hyperglycemia" is defined as the condition in which a subject has a fasting blood glucose concentration above the normal range, greater than 100 mg/dL (5.6 mmol/L). The word "fasting" has the usual meaning as a medical term.

The term **"hypoglycemia"** is defined as the condition in which a subject has a blood glucose concentration below the normal range, in particular below 70 mg/dL (3.89
mmol/L).

The term **"postprandial hyperglycemia"** is defined as the condition in which a subject has a 2 hour postprandial blood glucose or serum glucose concentration greater than 200 mg/dL (11.11 mmol/L).

The term **"impaired fasting blood glucose" or "IFG"** is defined as the condition in which a subject has a fasting blood glucose concentration or fasting serum glucose concentration in a range from 100 to 125 mg/dl (i.e. from 5.6 to 6.9 mmol/l), in particular greater than 110 mg/dL and less than 126 mg/dl (7.00 mmol/L). A subject with "normal fasting glucose" has a fasting glucose concentration smaller than 100 mg/dl, i.e. smaller than 5.6 mmol/l.

The term **"impaired glucose tolerance" or "IGT"** is defined as the condition in which a subject has a 2 hour postprandial blood glucose or serum glucose concentration greater than 140 mg/dl (7.78 mmol/L) and less than 200 mg/dL (11.11 mmol/L). The abnormal glucose tolerance, i.e. the 2 hour postprandial blood glucose or serum glucose concentration can be measured as the blood sugar level in mg of glucose per dL of plasma 2 hours after taking 75 g of glucose after a fast. A subject with "normal glucose tolerance" has a 2 hour postprandial blood glucose or serum glucose concentration smaller than 140 mg/dl (7.78 mmol/L).

The term **"hyperinsulinemia"** is defined as the condition in which a subject with insulin resistance, with or without euglycemia, has fasting or postprandial serum or plasma insulin concentration elevated above that of normal, lean individuals without insulin resistance, having a waist-to-hip ratio < 1.0 (for men) or < 0.8 (for women).

The terms "insulin-sensitizing", "insulin resistance-improving" or "insulin resistance-lowering" are synonymous and used interchangeably.

The term **"insulin resistance"** is defined as a state in which circulating insulin levels in excess of the normal response to a glucose load are required to maintain the euglycemic state (Ford ES, et al. JAMA. (2002) 287:356-9). A method of determining insulin resistance is the euglycaemic-hyperinsulinaemic clamp test. The ratio of insulin to glucose is determined within the scope of a combined insulin-glucose infusion technique. There is found to be insulin resistance if the glucose absorption is below the 25th percentile of the background population investigated (WHO definition). Rather less laborious than the clamp test are so called minimal models in which, during an intravenous glucose tolerance test, the insulin and glucose concentrations in the blood are measured at fixed time intervals and from these the insulin resistance is calculated. With this method, it is not possible to distinguish between hepatic and peripheral insulin resistance.

Furthermore, insulin resistance, the response of a patient with insulin resistance to therapy, insulin sensitivity and hyperinsulinemia may be quantified by assessing the "homeostasis model assessment to insulin resistance (HOMA-IR)" score, a reliable indicator of insulin resistance (Katsuki A, et al. Diabetes Care 2001; 24: 362-5). Further reference is made to methods for the determination of the HOMA-index for insulin sensitivity (Matthews et al., Diabetologia 1985, 28: 412-19), of the ratio of intact proinsulin to insulin (Forst et al., Diabetes 2003, 52(Suppl.1): A459*)* and to an euglycemic clamp study. In addition, plasma adiponectin levels can be monitored as a potential surrogate of insulin sensitivity. The estimate of insulin resistance by the homeostasis assessment model (HOMA)-IR score is calculated with the formula (Galvin P, et al. Diabet Med 1992;9:921-8):$HOMA - IR = \left(fasting serum insulin\left(μU/mL\right)\right) \times \left[fasting plasma glucose\left(mmol/L\right)/22.5\right]$

Insulin resistance can be confirmed in these individuals by calculating the HOMA-IR score. For the purpose of this invention, insulin resistance is defined as the clinical condition in which an individual has a HOMA-IR score > 4.0 or a HOMA-IR score above the upper limit of normal as defined for the laboratory performing the glucose and insulin assays.

As a rule, other parameters are used in everyday clinical practice to assess insulin resistance. Preferably, the patient's triglyceride concentration is used, for example, as increased triglyceride levels correlate significantly with the presence of insulin resistance.

Individuals likely to have insulin resistance are those who have two or more of the following attributes: 1) overweight or obese, 2) high blood pressure, 3) hyperlipidemia, 4) one or more 1^{st} degree relative with a diagnosis of IGT or IFG or type 2 diabetes.

Patients with a predisposition for the development of IGT or IFG or type 2 diabetes are those having euglycemia with hyperinsulinemia and are by definition, insulin resistant. A typical patient with insulin resistance is usually overweight or obese. If insulin resistance can be detected, this is a particularly strong indication of the presence of pre-diabetes. Thus, it may be that in order to maintain glucose homoeostasis a person needs 2-3 times as much insulin as a healthy person, without this resulting in any clinical symptoms.

**"Pre-diabetes"** is a general term that refers to an intermediate stage between normal glucose tolerance (NGT) and overt type 2 diabetes mellitus (T2DM), also referred to as intermediate hyperglycaemia. Therefore in one aspect of the present invention "pre-diabetes" is diagnosed in an individual if HbA1c is more or equal to 5.7% and less than 6.5%. According to another aspect of this invention "pre-diabetes" represents 3 groups of individuals, those with impaired glucose tolerance (IGT) alone, those with impaired fasting glucose (IFG) alone or those with both IGT and IFG. IGT and IFG usually have distinct pathophysiologic etiologies, however also a mixed condition with features of both can exist in patients. Therefore in another aspect of the present invention a patient being diagnosed of having "pre-diabetes" is an individual with diagnosed IGT or diagnosed IFG or diagnosed with both IGT and IFG. Following the definition according to the American Diabetes Association (ADA) and in the context an aspect of the present invention a patient being diagnosed of having "pre-diabetes" is an individual with:
a) a fasting plasma glucose (FPG) concentration <100 mg/dL [1 mg/dL = 0.05555 mmol/L] and a 2-hour plasma glucose (PG) concentration, measured by a 75-g oral glucose tolerance test (OGTT), ranging between ≥140 mg/dL and <200 mg/dL (i.e., IGT); or
b) a fasting plasma glucose (FPG) concentration between ≥100 mg/dL and <126 mg/dL and a 2-hour plasma glucose (PG) concentration, measured by a 75-g oral glucose tolerance test (OGTT) of <140 mg/dL (i.e., IFG); or
c) a fasting plasma glucose (FPG) concentration between ≥100 mg/dL and <126 mg/dL and a 2-hour plasma glucose (PG) concentration, measured by a 75-g oral glucose tolerance test (OGTT), ranging between ≥140 mg/dL and <200 mg/dL (i.e., both IGT and IFG).

Patients with "pre-diabetes" are individuals being pre-disposed to the development of type 2 diabetes. Pre-diabetes extends the definition of IGT to include individuals with a fasting blood glucose within the high normal range ≥ 100 mg/dL (J. B. Meigs, et al. Diabetes 2003; 52:1475-1484). The scientific and medical basis for identifying pre-diabetes as a serious health threat is laid out in a Position Statement entitled "The Prevention or Delay of Type 2 Diabetes" issued jointly by the American Diabetes Association and the National Institute of Diabetes and Digestive and Kidney Diseases (Diabetes Care 2002; 25:742-749).

The methods to investigate the **function of pancreatic beta-cells** are similar to the above methods with regard to insulin sensitivity, hyperinsulinemia or insulin resistance: An improvement of beta-cell function can be measured for example by determining a HOMA-index (homeostasis model assessment) for beta-cell function, HOMA-B, (Matthews et al., Diabetologia 1985, 28: 412-19), the ratio of intact proinsulin to insulin (Forst et al., Diabetes 2003, 52(Suppl.1): A459*),* first and second phase insulin secretion after an oral glucose tolerance test or a meal tolerance test (Stumvoll et al., Diabetes care 2000, 23: 295-301), the insulin/C-peptide secretion after an oral glucose tolerance test or a meal tolerance test, or by employing a hyperglycemic clamp study and/or minimal modeling after a frequently sampled intravenous glucose tolerance test (Stumvoll et al., Eur J Clin Invest 2001, 31: 380-81)*.*

The term **"type 1 diabetes"** is defined as the condition in which a subject has, in the presence of autoimmunity towards the pancreatic beta-cell or insulin, a fasting blood glucose or serum glucose concentration greater than 125 mg/dL (6.94 mmol/L). If a glucose tolerance test is carried out, the blood sugar level of a diabetic will be in excess of 200 mg of glucose per dL (11.1 mmol/l) of plasma 2 hours after 75 g of glucose have been taken on an empty stomach, in the presence of autoimmunity towards the pancreatic beta cell or insulin. In a glucose tolerance test 75 g of glucose are administered orally to the patient being tested after 10-12 hours of fasting and the blood sugar level is recorded immediately before taking the glucose and 1 and 2 hours after taking it. The presence of autoimmunity towards the pancreatic beta-cell may be observed by detection of circulating islet cell autoantibodies ["type 1A diabetes mellitus"], i.e., at least one of: GAD65 [glutamic acid decarboxylase-65], ICA [islet-cell cytoplasm], IA-2 [intracytoplasmatic domain of the tyrosine phosphatase-like protein IA-2], ZnT8 [zinc-transporter-8] or anti-insulin; or other signs of autoimmunity without the presence of typical circulating autoantibodies [type 1B diabetes], i.e. as detected through pancreatic biopsy or imaging). Typically a genetic predisposition is present (e.g. HLA, *INS* VNTR and *PTPN22),* but this is not always the case.

The term **"type 2 diabetes mellitus"** or **"T2DM"** is defined as the condition in which a subject has a fasting blood glucose or serum glucose concentration greater than 125 mg/dL (6.94 mmol/L). The measurement of blood glucose values is a standard procedure in routine medical analysis. If a glucose tolerance test is carried out, the blood sugar level of a diabetic will be in excess of 200 mg of glucose per dL (11.1 mmol/l) of plasma 2 hours after 75 g of glucose have been taken on an empty stomach. In a glucose tolerance test 75 g of glucose are administered orally to the patient being tested after 10-12 hours of fasting and the blood sugar level is recorded immediately before taking the glucose and 1 and 2 hours after taking it. In a healthy subject, the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg, this is regarded as abnormal glucose tolerance.

The term **"late stage type 2 diabetes mellitus"** includes patients with a secondary drug failure, indication for insulin therapy and progression to micro- and macrovascular complications e.g. diabetic nephropathy, or coronary heart disease (CHD).

The term **"LADA"** ("latent autoimmune diabetes of adults") refers to patients that have a clinical diagnosis of type 2 diabetes, but who are being detected to have autoimmunity towards the pancreatic beta cell. Latent autoimmune diabetes of adults (LADA) is also known as slowly progressive type 1 diabetes mellitus (T1DM), "mild" T1DM, non-insulin dependent type 1 DM, type 1 ½ DM, double diabetes or antibody positive type 2 DM (T2DM). LADA is often not clearly defined and, opposed to T1DM, seldom or never presents with significant weight loss and ketoacidosis due to rapidly progressive β-cell failure.

The term **"HbA1c"** refers to the product of a non-enzymatic glycation of the haemoglobin B chain. Its determination is well known to one skilled in the art. In monitoring the treatment of diabetes mellitus the HbA1c value is of exceptional importance. As its production depends essentially on the blood sugar level and the life of the erythrocytes, the HbA1c in the sense of a "blood sugar memory" reflects the average blood sugar levels of the preceding 4-6 weeks. Diabetic patients whose HbA1c value is consistently well adjusted by intensive diabetes treatment (i.e. < 6.5 % of the total haemoglobin in the sample), are significantly better protected against diabetic microangiopathy. For example, metformin on its own achieves an average improvement in the HbA1c value in the diabetic of the order of 1.0 - 1.5 %. This reduction of the HbA1C value is not sufficient in all diabetics to achieve the desired target range of <7% or < 6.5 % and preferably < 6 % HbA1c.

The term **"insufficient glycemic control"** or "inadequate glycemic control" in the scope of the present invention means a condition wherein patients show HbA1c values above 6.5 %, in particular above 7.0 %, even more preferably above 7.5 %, especially above 8 %.

The **"metabolic syndrome",** also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel III) JAMA: Journal of the American Medical Association (2001) 285:2486-2497), diagnosis of the metabolic syndrome is made when three or more of the following risk factors are present:
1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women;
2. Triglycerides: ≥ 150 mg/dL
3. HDL-cholesterol < 40 mg/dL in men
4. Blood pressure ≥ 130/85 mm Hg (SBP ≥ 130 or DBP ≥ 85)
5. Fasting blood glucose ≥ 100 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/Main, 2000.

According to a commonly used definition, **hypertension** is diagnosed if the systolic blood pressure (SBP) exceeds a value of 140 mm Hg and diastolic blood pressure (DBP) exceeds a value of 90 mm Hg. If a patient is suffering from manifest diabetes it is currently recommended that the systolic blood pressure be reduced to a level below 130 mm Hg and the diastolic blood pressure be lowered to below 80 mm Hg.

The term **"empagliflozin"** refers to the SGLT2 inhibitor 1-chloro-4-(β-D-glucopyranos-1-yl)-2-[4-((*S)-*tetrahydrofuran-3-yloxy)-benzyl]-benzene of the formula 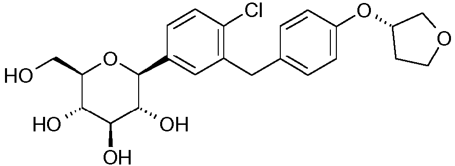 as described for example in WO 2005/092877. Methods of synthesis are described in the literature, for example WO 06/120208 and WO 2011/039108. According to this invention, it is to be understood that the definition of empagliflozin also comprises its hydrates, solvates and polymorphic forms thereof, and prodrugs thereof. An advantageous crystalline form of empagliflozin is described in WO 2006/117359 and WO 2011/039107 which hereby are incorporated herein in their entirety. This crystalline form possesses good solubility properties which enables a good bioavailability of the SGLT2 inhibitor. Furthermore, the crystalline form is physico-chemically stable and thus provides a good shelf-life stability of the pharmaceutical composition. Preferred pharmaceutical compositions, such as solid formulations for oral administration, for example tablets, are described in WO 2010/092126, which hereby is incorporated herein in its entirety.

The terms **"treatment"** and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

The terms **"prophylactically treating",** "preventivally treating" and "preventing" are used interchangeably and comprise a treatment of patients at risk to develop a condition mentioned hereinbefore, thus reducing said risk.

The term **"tablet"** comprises tablets without a coating and tablets with one or more coatings. Furthermore the "term" tablet comprises tablets having one, two, three or even more layers and press-coated tablets, wherein each of the beforementioned types of tablets may be without or with one or more coatings. The term "tablet" also comprises mini, melt, chewable, effervescent and orally disintegrating tablets.

The terms **"pharmacopoe"** and **"pharmacopoeias"** refer to standard pharmacopoeias such as the "USP 31-NF 26 through Second Supplement" (United States Pharmacopeial Convention) or the "European Pharmacopoeia 6.3" (European Directorate for the Quality of Medicines and Health Care, 2000-2009).

The term **"chronic kidney disease (CDK)"** is defined as abnormalities of kidney structure or function, present for more than three months, with implications for health. CKD is classified based on cause, GFR category, and albuminuria category (CGA).

CKD has been classified into 5 stages, where stage 1 is kidney damage with normal GFR (mL/min/1.73 m2 ) of 90 or above; stage 2 is kidney damage with a mild decrease in GFR (GFR 60-89); stage 3 is a moderate decrease in GFR (GFR 30-59); stage 4 is a severe decrease in GFR (GFR 15-29); and stage 5 is kidney failure (GFR <15 or dialysis). Stage 3 has been subdivided into stage 3A, which is a mild to moderate decrease in GFR (GFR 45-59), and stage 3B, which is a moderate to severe decrease in GFR (GFR 30-44).

The term **"albuminuria"** is defined as a condition wherein more than the normal amount of albumin is present in the urine. Albuminuria can be determined by the albumin excretion rate (AER) and/or the albumin-to-creatine ratio (ACR) in the urine (also refered to as UACR). Albuminuria categories in CKD are defined as follows:

| | | ACR (approximate equivalent) | | |
|---|---|---|---|---|
| Category | AER (mg/24 hours) | (mg/mmol) | (mg/g) | Terms |
| A1 | < 30 | < 3 | < 30 | Normal to mildly increased |
| A2 | 30-300 | 3-30 | 30-300 | Moderately increased |
| A3 | >300 | >30 | >300 | Severely increased |

Category A1 reflects no albuminuria, category A2 reflects microalbuminuria, category A3 reflects macroalbuminuria. The progression of category A1 usually leads to microalbuminuria (A2) but may also directly result in macroalbuminuria (A3). Progression of microalbuminuria (A2) results in macroalbuminuria (A3).

The term **"eGFR"** refers to the estimated glomerular filtration rate (GFR). The GFR describes the flow rate of filtered fluid through the kidney. The estimated GFR may be calculated based on serum creatinine values e.g. using the Chronic Kidney Disease Epidemiology Collaboration (CKD-EPI) equation (also abbreviated as (CKD-EPI)cr), the Cockcroft-Gault formula or the Modification of Diet in Renal Disease (MDRD) formula, which are all known in the art.

According to an aspect of this invention the estimated glomerular filtration rate (eGFR) is derived from serum creatinine values, age sex and race based on the CKD-EPI equation: 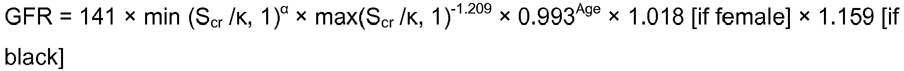 GFR = 141 × min (Scr /κ, 1)α × max(Scr /κ, 1)-1.209 × 0.993Age × 1.018 [if female] × 1.159 [if black] where:
Scr is serum creatinine in mg/dL,
κ is 0.7 for females and 0.9 for males,
α is -0.329 for females and -0.411 for males,
min indicates the minimum of S_{cr} /κ or 1, and
max indicates the maximum of S_{cr} /κ or 1.

For the purpose of the present invention, the degree of renal impairment in a patient is defined by the following estimated glomerular filtration rate (eGFR):
Normal renal function (CKD stage 1): eGFR ≥ 90 mL/min/1.73 m²
Mild renal impairment (CKD stage 2): eGFR ≥60 to <90 mL/min/1.73 m²
Moderate renal impairment (CKD stage 3): eGFR ≥30 to <60 mL/min/1.73 m²
Severe renal impairment (CKD stage 4): eGFR ≥15 to <30 mL/min/1.73 m²
Kidney failure (CKD stage 5): eGFR <15 mL/min/1.73 m²

According to the present invention moderate renal impairment can be further divided into two sub-stages:
Moderate A renal impairment (CKD 3A): eGFR ≥45 to <60 mL/min/1.73 m²
Moderate B renal impairment (CKD 3B): eGFR ≥30 to <45 mL/min/1.73 m²

### Detailed Description of the Invention

Beyond an improvement of glycemic control and weight loss due to an increase in urinary glucose excretion, empagliflozin shows a diuretic effect, reduced arterial stiffness and direct vascular effects (Cherney et al., Cardiovasc Diabetol. 2014;13:28; Cherney et al., Circulation. 2014;129:587-597). In the EMPA-REG OUTCOME^{™} study it was demonstrated that empagliflozin reduced the risk of cardiovascular death, hospitalization for heart failure and overall mortality in patients with type 2 diabetes mellitus and high cardiovascular risk (Zinman et al., N Engl J Med. 2015;373:2117-2128). It was observed that treatment with empagliflozin leads to blood pressure reductions without clinically relevant changes of the heart rate, thus improving rate pressure product (RPP), a surrogate marker of cardiac oxygen demand. Furthermore empagliflozin was found of not being associated with clinically relevant reflex-mediated sympathetic activation in contrast to increases observed with diuretics. It may be assumed that altered glucose and sodium gradients within the kidney may generate a sympathoinhibitory afferent renal nerve signal. The lack of sympathetic activation may contribute to a beneficial cardiovascular and renal profile of empagliflozin (cardiorenal axis). Based on clinical and non-clinical studies including mechanistic considerations, such as the effect of empagliflozin on human autonomic cardiovascular regulation, the use of empagliflozin in the treatment, prevention or slowing the progression of certain diseases and conditions or reducing the risk thereof, in particular chronic kidney disease and cardiovascular death in certain patients, is described hereinbefore and hereinafter.

According to one embodiment, this invention provides a method of treating, reducing the risk of or slowing the progression of chronic kidney disease in a patient with chronic kidney disease, said method comprising administering empagliflozin to the patient.

According to one embodiment, this invention provides a method for treating, preventing, protecting against, reducing the risk of, delaying the occurrence of and/or delaying the progression of chronic kidney disease in a patient not diagnosed with chronic heart failure comprising administering empagliflozin to the patient wherein the patient is a non-diabetic patient. In particular this embodiment relates to a method for treating and/or delaying the progression of chronic kidney disease in the patient. According to an aspect of this embodiment the patient is a patient with stage 3, including stage 3a and/or 3b, chronic kidney disease. According to another aspect of this embodiment the patient is a patient with stage 4 chronic kidney disease.

Chronic kidney disease (CKD), also known as chronic renal disease, is a progressive loss in renal function over a period of months or years. Patients with renal disease, renal dysfunction or renal impairment may include patients with chronic renal insufficiency or impairment, which can be stratified (if not otherwise noted) according to glomerular filtration rate (GFR, ml/min/1.73m²) into 5 disease stages: stage 1 characterized by normal GFR ≥ 90 plus either persistent albuminuria (e.g. UACR ≥30 mg/g) or known structural or hereditary renal disease; stage 2 characterized by mild reduction of GFR (GFR 60-89) describing mild renal impairment; stage 3 characterized by moderate reduction of GFR (GFR 30-59) describing moderate renal impairment; stage 4 characterized by severe reduction of GFR (GFR 15-29) describing severe renal impairment; and terminal stage 5 characterized by requiring dialysis or GFR < 15 describing established kidney failure (end-stage renal disease, ESRD).

Chronic kidney disease and its stages (CKD 1-5) can be usually characterized or classified accordingly, such as based on the presence of either kidney damage (albuminuria) or impaired estimated glomerular filtration rate (GFR <60 [ml/min/1.73m²], with or without kidney damage).

Generally, mild renal impairment according to the present invention corresponds to stage 2 chronic kidney disease, moderate renal impairment according to the present invention generally corresponds to stage 3 chronic kidney disease, and severe renal impairment according to the present invention generally corresponds to stage 4 chronic kidney disease. Likewise, moderate A renal impairment according to the present invention generally corresponds to stage 3A chronic kidney disease and moderate B renal impairment according to the present invention generally corresponds to stage 3B chronic kidney disease.

According to an embodiment, this invention provides a method for reducing the risk of cardiovascular death in a patient with chronic kidney disease.

According to an embodiment, this invention provides a method for reducing the risk of all-cause mortality in a patient with chronic kidney disease.

According to an embodiment, this invention provides a method for reducing the risk of hospitalization in a patient with chronic kidney disease.

According to an embodiment, this invention provides a method for reducing the risk of heart failure hospitalization in a patient with chronic kidney disease.

According to an embodiment, this invention provides a method for reducing the risk of all cause hospitalization in a patient with chronic kidney disease.

According to an embodiment this invention provides a method for reducing the risk of any of cardiovascular death (including fatal stroke, fatal myocardial infarction and sudden death), non-fatal myocardial infarction (excluding silent myocardial infarction), non-fatal stroke (the so-called 3-point MACE) in a patient with chronic kidney disease.

In another embodiment, the present invention provides a method of preventing, reducing the risk of or delaying the occurrence of a cardiovascular event, said method comprising administering empagliflozin, optionally in combination with one or more other therapeutic substances, to the CKD patient. In one embodiment, the cardiovascular event is selected from cardiovascular death, non-fatal myocardial infarction, non-fatal stroke, hospitalisation for unstable angina pectoris and heart failure requiring hospitalisation. In one embodiment, the cardiovascular death is due to fatal myocardial infarction or fatal stroke. In one embodiment, the cardiovascular death is due to sudden death or heart failure death.

In one embodiment, the present invention provides a method of treating, reducing the risk of or slowing the progression of chronic kidney disease and reducing the risk of cardiovascular death in a patient with chronic kidney disease, the method comprising administering empagliflozin to the patient. In one aspect the patient has moderately or severely decreased renal function or elevated albuminuria levels, for example ≥200 mg/g.

In one aspect, the patient has an eGFR ≥20 to <45 mL/min/1.73 m². In one aspect, the patient has an eGFR ≥20 mL/min/1.73 m² and a urinary albumin-to-creatine ratio (UACR) ≥200 mg/g. In one aspect, the patient has an eGFR ≥45 and <90 ml/min/1.73m² and a urinary albumin-to-creatine ratio (UACR) ≥ 200mg/g.

In one aspect, the patient is treated with a RAAS inhibitor (Renin-Angiotensin-Aldosterone System). In one aspect, the patient is treated with an Angiotensin-Converting Enzyme (ACE) inhibitor and/or an angiotensin II receptor blocker (ARB).In one aspect, the patient is on standard of care according to local/international guideline to treat CKD risk factors.

In one aspect, the patient is a non-diabetic patient. In one aspect, the patient is a patient with pre-diabetes or a patient with type 2 or type 1 diabetes mellitus.

In one aspect, the patient is a non-diabetic and non-pre-diabetic patient. In one aspect, the patient is not at risk or even high risk for cardiovascular events. In one aspect, the patient is a not a patient with chronic heart failure, in particular not a patient with HFrEF (Heart Failure with reduced Ejection Fraction) and/or HFpEF (Heart Failure with preserved Ejection Fraction).

In one aspect, empagliflozin is administered at a dose in a range from 1 mg to 25 mg, for example at a dose of 10 mg or 25 mg. In one aspect, empagliflozin is administered once daily to the patient.

In one aspect, empagliflozin slows or delays the time to the first occurrence of any of the following components:
- CV death
- sustained decrease of 40% or more in eGFR, e.g. via (CKD-EPI)cr
- incidence of ESRD defined by either
   ∘ continuous renal replacement therapy (dialysis or renal transplantation) or
   ∘ sustained eGFR <15 ml/min/1.73m² for patients with baseline eGFR ≥30 ml/min/1.73m² or eGFR <10 ml/min/1.73m² for patients with baseline eGFR <30 ml/min/1.73m²

In one aspect, empagliflozin slows or delays the time to the first occurrence of renal disease progression as defined by one or more of the following:
∘ sustained decrease of 40% or more in eGFR, e.g. via (CKD-EPI)cr
∘ sustained eGFR <10 ml/min/1.73m²
∘ Incidence of ESRD defined by continuous renal replacement therapy (by either dialysis or renal transplantation) or
∘ renal death

In one aspect, empagliflozin slows or delays the time to the first occurrence of any of the following components: Occurrence of
- All-cause mortality or all-cause hospitalization
- All-cause hospitalization
- All-cause mortality
- eGFR (e.g. via (CKD-EPI)cr) slope of change from baseline
- Cardiovascular death or hospitalization for heart failure

In one aspect, empagliflozin slows or delays the time to any one of the following components:
- Time to CV death confirmed by adjudication
- Time to first occurrence of sustained decrease of 40% or more in eGFR confirmed by adjudication
- Time to first incidence of ESRD confirmed by adjudication
- Time to first hospitalization for congestive heart failure confirmed by adjudication
- Time to first all-cause hospitalization
- Time to all-cause mortality
- Time to first 3-MACE (i.e. CV death, non-fatal MI, non-fatal stroke) confirmed by adjudication
- Time to first occurrence of all-cause mortality, sustained decrease of 40% or more in eGFR or incidence of ESRD confirmed by adjudication
- Time to first occurrence of all-cause mortality, sustained decrease of 50% or more in eGFR or incidence of ESRD confirmed by adjudication
- Time to first occurrence of all-cause mortality, sustained decrease of 57% or more in eGFR or incidence of ESRD confirmed by adjudication
- Time to first occurrence of composite renal endpoint (sustained decrease of 40% or more in eGFR, incidence of ESRD) confirmed by adjudication
- Time to first occurrence of composite renal endpoint 2 (sustained decrease of 50% or more in eGFR, incidence of ESRD) confirmed by adjudication
- Time to first occurrence of composite renal endpoint 3 (sustained decrease of 57% or more in eGFR, incidence of ESRD) confirmed by adjudication
- Time to first sustained decrease of 57% or more in eGFR confirmed by adjudication
- Time to first sustained decrease of 50% or more in eGFR confirmed by adjudication
- Time to first sustained decrease of 30% or more in eGFR confirmed by adjudication
- Time to first incidence of acute renal failure (incl. AKI) confirmed by adjudication
- Time to first incidence of AKI confirmed by adjudication
- Time to onset of DM (defined as HbA1c ≥6.5% or as diagnosed by the Investigator) in patients without DM defined as no history of DM and HbA1c <6.5% at baseline

In the methods according to the present invention empagliflozin is optionally administered in combination with one or more other therapeutic substances to the patient.

According to an embodiment of the methods as described hereinbefore and hereinafter the patient is a non-diabetic patient, a patient with pre-diabetes, a patient with type 2 diabetes mellitus or a patient with type 1 diabetes mellitus.

According to another embodiment of the methods as described hereinbefore and hereinafter the patient is a patient with pre-diabetes. According to an aspect of this embodiment the patient has a HbA1c more or equal to 5.7% and less than 6.5%.

According to another embodiment of the methods as described hereinbefore and hereinafter the patient is a patient with pre-diabetes or a non-diabetic patient. According to an aspect of this embodiment the patient has a HbA1c less than 6.5%.

According to another embodiment of the methods as described hereinbefore and hereinafter the patient is a non-diabetic patient, in particular a non-diabetic and non-pre-diabetic patient. According to an aspect of this embodiment the patient has a HbA1c less than 5.7%.

According to another aspect the non-diabetic patient does not show an impaired glucose tolerance (IGT), i.e. the patient shows a normal glucose tolerance. For example the 2 hour postprandial blood glucose or plasma glucose (PG) concentration is smaller than 140 mg/dl (7.78 mmol/L).

According to another aspect the non-diabetic patient does not show an impaired fasting blood glucose (IFG), i.e. the patient shows a normal fasting glucose. For example the fasting plasma glucose concentration (FPG) is smaller than 100 mg/dl, i.e. smaller than 5.6 mmol/l.

In particular the non-diabetic patient does not show an impaired fasting blood glucose (IFG) and does not show an impaired glucose tolerance (IGT), i.e. the patient shows a normal glucose tolerance and a normal glucose tolerance. For example the fasting plasma glucose concentration (FPG) is smaller than 100 mg/dl, i.e. smaller than 5.6 mmol/l, and the 2 hour postprandial blood glucose or plasma glucose (PG) concentration is smaller than 140 mg/dl (7.78 mmol/L).

According to an embodiment of the methods as described hereinbefore and hereinafter empagliflozin is administered at a dose in a range from 0.5 to 25 mg per day, for example 1 to 25 mg per day, for example at a dose of 1 mg, 2.5 mg, 5 mg, 7.5 mg, 10 mg or 25 mg per day to the patient. The administration of empagliflozin may occur one or two times a day, most preferably once a day. For example a dose for once daily administration is 10 mg or 25 mg. The preferred route of administration is oral administration.

According to a particular aspect of the present invention empagliflozin is administered at a dose of 10 mg per day to the patient.

According to another particular aspect of the present invention empagliflozin is administered at a dose of 25 mg per day to the patient.

Preferably empagliflozin is administered orally to the patient once daily.

In one embodiment, patients within the meaning of this invention may include patients with chronic heart failure who have not previously been treated with a drug to treat chronic heart failure (heart-failure-drug-naïve patients). Thus, in an embodiment, the therapies described herein may be used in heart-failure-drug-naïve patients.

In another embodiment, patients within the meaning of this invention may include patients with chronic heart failure and with pre-diabetes or with type 2 diabetes mellitus (T2DM) who have not previously been treated with an antidiabetic drug (T2DM-drug-naïve patients). Thus, in an embodiment, the therapies described herein may be used in T2DM-drug-naïve patients.

Furthermore, the methods according to this invention are particularly suitable in the treatment of patients with chronic heart failure and with insulin dependency, i.e. in patients who are treated or otherwise would be treated or need treatment with an insulin or a derivative of insulin or a substitute of insulin or a formulation comprising an insulin or a derivative or substitute thereof. These patients include patients with diabetes type 2 and patients with diabetes type 1.

Furthermore, it can be found that the administration of a pharmaceutical composition according to this invention results in no risk or in a low risk of hypoglycemia. Therefore, a treatment or prophylaxis according to this invention is also advantageously possible in those patients showing or having an increased risk for hypoglycemia.

By the administration of empagliflozin excessive blood glucose is excreted through the urine of the patient based on the SGLT2 inhibiting activity, so that no gain in weight or even a reduction in body weight of the patient may result. Therefore, the methods according to this invention are advantageously suitable in those patients with chronic heart failure who are diagnosed of one or more of the conditions selected from the group consisting of overweight and obesity, in particular class I obesity, class II obesity, class III obesity, visceral obesity and abdominal obesity. In addition a method according to this invention is advantageously suitable in those patients in which a weight increase is contraindicated.

When this invention refers to patients requiring treatment or prevention, it relates primarily to treatment and prevention in humans, but the pharmaceutical composition may also be used accordingly in veterinary medicine in mammals. In the scope of this invention adult patients are preferably humans of the age of 18 years or older. Also in the scope of this invention, patients are adolescent humans, i.e. humans of age 6 to 17 years, for example 10 to 17 years, preferably of age 13 to 17 years.

According to an embodiment of the present invention empagliflozin is administered in combination with one or more other therapeutic substances to the patient. The combined administration may be simultaneously, separately or sequentially.

In one embodiment, the active substances that are indicated in the treatment of chronic heart failure are selected from angiotensin receptor blockers (ARB), angiotensin-converting enzyme (ACE) inhibitors, angiotensin receptor neprilysin inhibitors (ARNi), beta-blockers, aldosterone antagonists (MRA), digoxin, ivabradine and diuretics.

In one embodiment, the antidiabetic substances are selected from metformin, sulphonylureas, nateglinide, repaglinide, PPAR-gamma agonists, alpha-glucosidase inhibitors, insulin and insulin analogues, GLP-1 and GLP-1 analogues and DPP-4 inhibitors.

In one embodiment the patient receives standard of care medication indicated for patients with chronic heart failure. In one aspect of this embodiment empagliflozin is administered to the patient in combination with one or more active substances that are indicated in the treatment of chronic heart failure. For example empagliflozin is adminstered in combination with one or more active substances selected from the group consisting of angiotensin receptor blockers (ARB), angiotensin-converting enzyme (ACE) inhibitors, beta-blockers, aldosterone antagonists, diuretics, angiotensin receptor-neprilysin inhibitor (ARNi), mineralcorticoid receptor antagonists and ivabradine. According to this aspect of the embodiment the patient is for example a non-diabetic patient or a patient with pre-diabetes.

Examples of angiotensin II receptor blockers (ARBs) are telmisartan, candesartan, valsartan, losartan, irbesartan, olmesartan, azilsartan and eprosartan; the dosage(s) of some of these medications are for example shown below:
- Candesartan (Atacand), 4 mg, 8 mg, 16 mg, or 32 mg of candesartan cilexetil
- Eprosartan (Teveten), 400 mg or 600 mg
- Irbesartan (Avapro), 75 mg, 150mg, or 300 mg of irbesartan.
- Losartan (Cozaar), 25 mg, 50 mg or 100 mg of losartan potassium
- Telmisartan (Micardis) , 40 mg or 80 mg
- Telmisartan (Micardis HCT) , 40 mg/12.5 mg, 80 mg/12.5 mg, and 80 mg/25 mg each of telmisartan and hydrochlorothiazide
- Telmisartan/amlodipine (Twynsta) , 40 mg/5 mg, 40 mg/10 mg, 80 mg/5 mg and 80 mg/ 10 mg each of telmisartan and amlodipine
- Valsartan (Diovan) , 40 mg, 80 mg, 160 mg or 320 mg of valsartan

Examples of Angiotensin-Converting Enzyme (ACE) inhibitors are benazepril, captopril, ramipril, lisinopril, Moexipril, cilazapril, quinapril, captopril, enalapril, benazepril, perindopril, fosinopril and trandolapril; the dosage(s) of some of these medications are for example shown below:
- Benazepril (Lotensin), 5 mg, 10 mg, 20 mg, and 40 mg for oral administration
- Captopril (Capoten), 12.5 mg, 25 mg, 50 mg, and 100 mg as scored tablets for oral administration
- Enalapril (Vasotec), 2.5 mg, 5 mg, 10 mg, and 20 mg tablets for oral administration
- Fosinopril (Monopril), for oral administration as 10 mg, 20 mg, and 40 mg tablets
- Lisinopril (Prinivil, Zestril), 5 mg, 10 mg, and 20 mg tablets for oral administration
- Moexipril (Univasc), 7.5 mg and 15 mg for oral administration
- Perindopril (Aceon), 2 mg, 4 mg and 8 mg strengths for oral administration
- Quinapril (Accupril), 5 mg, 10 mg, 20 mg, or 40 mg of quinapril for oral administration
- Ramipril (Altace), 1.25 mg, 2.5 mg, 5, mg, 10 mg
- Trandolapril (Mavik) , 1 mg, 2 mg, or 4 mg of trandolapril for oral administration

Examples of beta-blockers are acebutolol, atenolol, betaxolol, bisoprolol, celiprolol, metoprolol, nebivolol, propranolol, timolol and carvedilol; the dosage(s) of some of these medications are for example shown below:
- Acebutolol (Sectral), 200 or 400 mg of acebutolol as the hydrochloride salt
- Atenolol (Tenormin), 25, 50 and 100 mg tablets for oral administration
- Betaxolol (Kerlone), 10-mg and 20-mg tablets for oral administration
- Bisoprolol/hydrochlorothiazide (Ziac), 2.5/6 mg, 5/6.25 mg, 10/6.25 mg
- Bisoprolol (Zebeta), 5 and 10 mg tablets for oral administration
- Metoprolol (Lopressor, Toprol XL), 50- and 100-mg tablets for oral administration and in 5-mL ampuls for intravenous administration
- Propranolol (Inderal), 10 mg, 20 mg, 40 mg, 60 mg, and 80 mg tablets for oral administration
- Timolol (Blocadren), 5 mg, 10 mg or 20 mg timolol maleate for oral administration.

Examples of aldosterone antagonists are spironolactone, eplerenone, canrenone and fineronone; the dosage(s) of some of these medications are for example shown below:
- spironolactone (e.g. Aldactone), 25 or 50 mg once daily or every second day,
- eplerenone (e.g. Inspra), 25 or 50 mg once daily.

Examples of diuretics are bumetanide, hydrochlorothiazide, chlortalidon, chlorothiazide, hydrochlorothiazide, xipamide, indapamide, furosemide, piretanide, torasemide, spironolactone, eplerenone, amiloride and triamterene; for example these medications are thiazide diuretics, e.g. chlorthalidone, HCT, loop diuretics, e.g. furosemide, torasemide or potassium-sparing diuretics, e.g. eplerenone, or combination thereof; the dosage(s) of some of these medications are for example shown below:
- Amiloride (Midamor), 5 mg of anhydrous amiloride HCl
- Bumetanide (Bumex), available as scored tablets, 0.5 mg (light green), 1 mg (yellow) and 2 mg (peach) for oral administration
- Chlorothiazide (Diuril),
- Chlorthalidone (Hygroton)
- Furosemide (Lasix)
- Hydro-chlorothiazide (Esidrix, Hydrodiuril)
- Indapamide (Lozol) and Spironolactone (Aldactone)
- Eplerenone (Inspra)

An example of an angiotensin receptor-neprilysin inhibitor (ARNi) is a combination of valsartan and sacubitril (Entresto).

An example of inhibition of the cardiac pacemaker I_{f} current is ivabradine (Procoralan, Corlanor).

Examples of calcium channel blockers are amlodipine, nifedipine, nitrendipine, nisoldipine, nicardipine, felodipine, lacidipine, lercanipidine, manidipine, isradipine, nilvadipine, verapamil, gallopamil and diltiazem.

Examples of medications that lower blood pressure include angiotensin II receptor blockers (ARBs), Angiotensin-Converting Enzyme (ACE) inhibitors, beta-blockers, diuretics and calcium channel blockers.

In another aspect of this embodiment the patient is a patient with type 2 diabetes mellitus and empagliflozin is administered to the patient in combination with one or more active substances that are indicated in the treatment of chronic heart failure and in combination with one or more antidiabetic substances. The antidiabetic substances include metformin, sulphonylureas, nateglinide, repaglinide, PPAR-gamma agonists, alpha-glucosidase inhibitors, insulin and insulin analogues, GLP-1 and GLP-1 analogues and DPP-4 inhibitors. Examples thereof are metformin and DPPIV inhibitors, such as sitagliptin, saxaglitpin and linagliptin. The active substances that are indicated in the treatment of chronic heart failure include angiotensin receptor blockers (ARB), angiotensin-converting enzyme (ACE) inhibitors, beta-blockers, aldosterone antagonists and diuretics.

Therefore according to one aspect of the methods according to this invention empagliflozin is administered in combination with linagliptin to the patient. The patient according to this aspect is in particular a patient with type 2 diabetes mellitus. Preferred doses are for example 10 mg empagliflozin once daily and 5 mg linagliptin once daily.

Therefore according to another aspect of the methods according to this invention empagliflozin is administered in combination with metformin hydrochloride to the patient. The patient according to this aspect is in particular a patient with type 2 diabetes mellitus. Preferred doses are for example 10 mg empagliflozin once daily or 5 mg empagliflozin twice daily and 500 mg, 850 mg or 1000 mg metformin hydrochloride twice daily.

In one aspect of this embodiment, the number, dosage and/or regimen of said medications to treat chronic heart failure is reduced in said patient, while the administration of empagliflozin is continued. In another aspect of this embodiment, the number, dosage and/or regimen of said medications to treat type 2 diabetes mellitus is reduced in said patient, while the administration of empagliflozin is continued. In yet another aspect of this embodiment, the numbers, dosages and/or regimens of said medications to treat type 2 diabetes mellitus and of said medications to treat chronic heart failure are reduced in said patient, while the administration of empagliflozin is continued.

According to an example of this aspect empagliflozin is adminstered in combination with one or more active substances selected from the group consisting of angiotensin receptor blockers (ARB), angiotensin-converting enzyme (ACE) inhibitors, beta-blockers, aldosterone antagonists, diuretics, angiotensin receptor-neprilysin inhibitor (ARNi), mineralcorticoid receptor antagonists and ivabradine in combination with metformin or in combination with linagliptin or in combination of metformin and linagliptin.

Examples of active substances in the above described groups are known to the one skilled in the art, including their dose strengths, administration schemes and formulations.

In the context of this invention the term metformin comprises metformin hydrochloride in the form of an immediate release, extended or slow release formulation. Doses of metformin hydrochloride administered to the patient are particularly 500 mg to 2000 mg per day, for example 750 mg, 1000 mg, 1500 and 2000 mg per day.

Empagliflozin and metformin may be adminstered separately in two different dosage forms or combined in one dosage form. Combined dosage forms of empagliflozin and metformin as immediate release formulations are described in WO 2011/039337 and are known for example as SYNJARDI^{®}. Combined dosage forms of empagliflozin and metformin wherein empagliflozin is part of an immediate release formulation and metformin is part of an extended release formulation are described in WO 2012/120040 and WO 2013/131967.

A preferred dose of linagliptin administered to the patient is 5 mg per day.

Empagliflozin and linagliptin may be administered separately in two different dosage forms or combined in one dosage form. Combined dosage forms of empagliflozin and linagliptin are described in WO 2010/092124 and are known for example as GLYXAMBI^{®}.

Within this invention it is to be understood that the combinations, compositions or administrations in combination according to this invention may envisage the simultaneous, sequential or separate administration of the active components or ingredients.

In this context, "combination" or "combined" within the meaning of this invention may include, without being limited, fixed and non-fixed (e.g. free) forms (including kits) and uses, such as e.g. the simultaneous, sequential or separate use of the components or ingredients.

The combined administration of this invention may take place by administering the active components or ingredients together, such as e.g. by administering them simultaneously in one single or in two separate formulations or dosage forms. Alternatively, the administration may take place by administering the active components or ingredients sequentially, such as e.g. successively in two separate formulations or dosage forms.

For the combination therapy of this invention the active components or ingredients may be administered separately (which implies that they are formulated separately) or formulated altogether (which implies that they are formulated in the same preparation or in the same dosage form). Hence, the administration of one element of the combination of the present invention may be prior to, concurrent to, or subsequent to the administration of the other element of the combination.

Unless otherwise noted, combination therapy may refer to first line, second line or third line therapy, or initial or add-on combination therapy or replacement therapy.

The methods according to this invention are particularly suitable in the long term treatment or prophylaxis of the diseases and/or conditions as described hereinbefore and hereinafter. The term "long term" as used hereinbefore and hereinafter indicates a treatment of or administration in a patient within a period of time longer than 12 weeks, preferably longer than 25 weeks, even more preferably longer than 1 year.

The pharmaceutical composition comprising empagliflozin according to the invention may be formulated for oral or parenteral (including intramuscular, sub-cutaneous and intravenous) administration in liquid or solid form or in a form suitable for administration by inhalation or insufflation. Oral administration is preferred. The pharmaceutical composition may be formulated in the form of tablets, granules, fine granules, powders, capsules, caplets, soft capsules, pills, oral solutions, syrups, dry syrups, chewable tablets, troches, effervescent tablets, drops, suspension, fast dissolving tablets, oral fast-dispersing tablets, etc.. The pharmaceutical composition and the dosage forms preferably comprise one or more pharmaceutical acceptable carriers which must be "acceptable" in the sense of being compatible with the other ingredients of the formulation and not deleterious to the recipient thereof. Examples of pharmaceutically acceptable carriers are known to the one skilled in the art.

The pharmaceutical compositions and methods according to this invention show advantageous effects in the treatment and prevention of those diseases and conditions as described hereinbefore. Advantageous effects may be seen for example with respect to efficacy, dosage strength, dosage frequency, pharmacodynamic properties, pharmacokinetic properties, fewer adverse effects, convenience, compliance, etc..

Methods for the manufacture of empagliflozin are known to the one skilled in the art. Advantageously, the compounds according to this invention can be prepared using synthetic methods as described in the literature, including patent applications as cited hereinbefore. Preferred methods of manufacture are described in the WO 2006/120208 and WO 2007/031548. With regard to empagliflozin an advantageous crystalline form is described in the international patent application WO 2006/117359 which hereby is incorporated herein in its entirety.

Further embodiments, features and advantages of the present invention may become apparent from the following examples. The following examples serve to illustrate, by way of example, the principles of the invention without restricting it.

### Examples

### Example 1A: Treatment of patients with chronic kidney disease

Empagliflozin is administered to patients in a randomised, double-blind, placebo controlled, parallel group study to compare treatment with empagliflozin 10 mg once daily with placebo as add-on therapy to standard of care in patients with chronic kidney disease. The duration of the patients is preferably a long term treatment, for example between 30 and 48 months.

Patients include individuals with presence of chronic kidney disease with high risk of cardiorenal events defined by at least one of the following:
A) Very high levels of albuminuria (i.e. macroalbuminuria) defined as UACR ≥ 200mg/g creatinine and/or
B) Impaired renal function with estimated GFR <45 ml/min/1.73m².

Furthermore patients include those with stable single RAS blockade background therapy (i.e. either ACE-inhibitor or ARB with unchanged daily dose.

The composite primary endpoint of the study is time to the first occurrence of any of the following components:
- CV death
   ∘ sustained decrease of 40% or more in eGFR (CKD-EPI)cr
   ∘ incidence of ESRD defined by either
   ∘ continuous renal replacement therapy (dialysis or renal transplantation) or
   o sustained eGFR <15 ml/min/1.73m² for patients with baseline eGFR ≥30 ml/min/1.73m² or eGFR <10 ml/min/1.73m² for patients with baseline eGFR <30 ml/min/1.73m²

A secondary endpoint is defined as time to the first occurrence of any of the following components: Occurrence of
All-cause mortality or all-cause hospitalization
All-cause hospitalization
All-cause mortality
eGFR (CKD-EPI)cr slope of change from baseline

Other secondary endpoints are any one of the following components:
Time to CV death confirmed by adjudication
Time to first occurrence of sustained decrease of 40% or more in eGFR confirmed by adjudication
Time to first incidence of ESRD confirmed by adjudication
Time to first hospitalization for congestive heart failure confirmed by adjudication
Time to first all-cause hospitalization
Time to all-cause mortality
Time to first 3-MACE (i.e. CV death, non-fatal MI, non-fatal stroke) confirmed by adjudication
Time to first occurrence of all-cause mortality, sustained decrease of 40% or more in eGFR or incidence of ESRD confirmed by adjudication
Time to first occurrence of all-cause mortality, sustained decrease of 50% or more in eGFR or incidence of ESRD confirmed by adjudication
Time to first occurrence of all-cause mortality, sustained decrease of 57% or more in eGFR or incidence of ESRD confirmed by adjudication
Time to first occurrence of composite renal endpoint (sustained decrease of 40% or more in eGFR, incidence of ESRD) confirmed by adjudication
Time to first occurrence of composite renal endpoint 2 (sustained decrease of 50% or more in eGFR, incidence of ESRD) confirmed by adjudication
Time to first occurrence of composite renal endpoint 3 (sustained decrease of 57% or more in eGFR, incidence of ESRD) confirmed by adjudication
Time to first sustained decrease of 57% or more in eGFR confirmed by adjudication
Time to first sustained decrease of 50% or more in eGFR confirmed by adjudication
Time to first sustained decrease of 30% or more in eGFR confirmed by adjudication
Time to first incidence of acute renal failure (incl. AKI) confirmed by adjudication
Time to first incidence of AKI confirmed by adjudication
Time to onset of DM (defined as HbA1c ≥6.5% or as diagnosed by the Investigator) in patients without DM defined as no history of DM and HbA1c <6.5% at baseline

### Example 1B: Treatment of patients with chronic kidney disease

Empagliflozin is administered to patients in a randomised, double-blind, placebo controlled, parallel group study to compare treatment with empagliflozin 10 mg once daily with placebo as add-on therapy to standard of care in patients with chronic kidney disease. The duration of the patients is preferably a long term treatment, for example between 30 and 48 months.

Patients include individuals with presence of chronic kidney disease with high risk of cardiorenal events defined by at least one of the following:
A) Impaired renal function with estimated GFR ≥20 and <45 ml/min/1.73m² or
B) Estimated GFR ≥45 and <90 ml/min/1.73m² and very high levels of albuminuria (i.e. macroalbuminuria) defined as UACR ≥ 200mg/g creatinine

Furthermore patients include those with a clinically appropriate dose of single agent RAS blockade background therapy (i.e. either ACE-inhibitor or ARB). Those participants for whom RAS blockade is not considered indicated (e.g. due to concomitant medication or co-morbidity), or who cannot tolerate RAS blockade will still be eligible to enter the trial, but the reason for not using RAS blockade will be documented.

The composite primary endpoint of the study is time to the first occurrence of any of the following components:
- CV death
- Renal disease progression
   ∘ sustained decrease of 40% or more in eGFR (CKD-EPI)cr
   ∘ sustained eGFR <10 ml/min/1.73m²
   ∘ Incidence of ESRD defined by continuous renal replacement therapy (by either dialysis or renal transplantation) or
   ∘ renal death

The key secondary endpoint is defined as time to the first occurrence of any of the following components: Occurrence of
- Cardiovascular death or hospitalization for heart failure;
- Hospitalization from any cause; and
- All-cause mortality.

Other secondary outcomes which will include the individual components of the primary composite outcome:
- Renal disease progression (as defined above);
- Cardiovascular death.

Tertiary assessments will involve intention-to-treat analyses among all randomized participants of the effects of allocation to empagliflozin versus placebo during the scheduled treatment period on:
- Renal disease progression, overall and with ESRD and a sustained ≥40% decline in eGFR considered separately;
- Annual rate of change in eGFR, overall and separately from 2 months, in all participants and separately in various subdivisions (as specified below);
- Mortality from particular categories of causes, including cardiovascular (e.g. coronary death, sudden cardiac death [not know to be coronary], heart failure, other cardiac, stroke, and other vascular) and non-cardiovascular (e.g. renal, infection, cancer, other medical, and non-medical) causes;
- The primary outcome composite and separately, the annual rate of change in GFR, in various subdivisions based on assessments made at the Randomization visit:
   (a) History of prior disease (presence vs. absence): diabetes mellitus*; cardio-vascular disease; heart failure; peripheral arterial disease;
   (b) Participant characteristics: age, sex, region, blood pressure, body mass index;
   (c) Laboratory values: HbA1c; eGFR; urinary albumin:creatinine ratio; haematocrit;
   (d) Medication: RAS blockade; beta-blocker; diuretics;
- Major cardiovascular events (defined as the composite of cardiovascular death, myocardial infarction, stroke or hospitalization for heart failure);
- New-onset diabetes mellitus (defined as clinical diagnosis, commencement of glucose-lowering treatment, or central HbA1c ≥48 mmol/mol on at least one occasion) among participants without diabetes at baseline*, overall and separately among those with normoglycaemia or "pre-diabetes" (defined as HbA1c<39 [normoglycaemia] or ≥39 to <48 mmol/mol [pre-diabetes], respectively). *Diabetes at baseline is defined as patient-reported history of diabetes, use of glucose-lowering medication or baseline HbA1c ≥48 mmol/mol at Randomization visit.

### Example 2: Pharmaceutical Composition and Dosage Form

The following example of solid pharmaceutical compositions and dosage forms for oral administration serves to illustrate the present invention more fully without restricting it to the contents of the example. Further examples of compositions and dosage forms for oral administration, are described in WO 2010/092126. The term "active substance" denotes empagliflozin according to this invention, especially its crystalline form as described in WO 2006/117359 and WO 2011/039107.

Tablets containing 2.5 mg, 5 mg, 10 mg or 25 mg of the active substance empagliflozin. Amounts of the ingredients are provided in mg per film-coated tablet.

| **Active substance** | 2.5 mg/ per tablet | 5 mg/ per tablet | 10 mg/ per tablet | 25 mg/ per tablet |
|---|---|---|---|---|
| **Wet granulation** | | | | |
| Empagliflozin | 2.5000 | 5.000 | 10.00 | 25.00 |
| Lactose Monohydrate | 40.6250 | 81.250 | 162.50 | 113.00 |
| Microcrystalline Cellulose | 12.5000 | 25.000 | 50.00 | 40.00 |
| Hydroxypropyl Cellulose | 1.8750 | 3.750 | 7.50 | 6.00 |
| Croscarmellose Sodium | 1.2500 | 2.500 | 5.00 | 4.00 |
| Purified Water | q.s. | q.s. | q.s. | q.s. |

| **Dry Adds** | | | | |
|---|---|---|---|---|
| Microcrystalline Cellulose | 3.1250 | 6.250 | 12.50 | 10.00 |
| Colloidal silicon dioxide | 0.3125 | 0.625 | 1.25 | 1.00 |
| Magnesium stearate | 0.3125 | 0.625 | 1.25 | 1.00 |
| Total core | 62.5000 | 125.000 | 250.00 | 200.00 |

| **Film Coating** | | | | |
|---|---|---|---|---|
| Film coating system | 2.5000 | 4.000 | 7.00 | 6.00 |
| Purified Water | q.s. | q.s. | q.s. | q.s. |
| **Total** | **65.000** | **129.000** | **257.00** | **206.00** |

Details regarding the manufacture of the tablets, the active pharmaceutical ingredient, the excipients and the film coating system are described in WO 2010/092126, in particular in the Examples 5 and 6, which hereby is incorporated herein in its entirety.

## Claims

1. A method of treating, reducing the risk of or slowing the progression of chronic kidney disease in a patient with chronic kidney disease, said method comprising administering empagliflozin to the patient.

2. The method of claim 1, wherein the method additionally reduces the risk of cardiovascular death in the patient.

3. The method of claim 1, wherein the method additionally reduces the risk of all-cause mortality in the patient.

4. The method of claim 1, wherein the method additionally reduces the risk of all-cause hospitalization in the patient.

5. The method of any one of claims 1 to 4, wherein the patient has moderately to severely decreased renal function.

6. The method according to any one of claims 1 to 5, wherein the patient has elevated albuminuria levels ≥200 mg/g.

7. The method according to any one of claims 1 to 6, wherein the patient has an eGFR ≥20 to <45 mL/min/1.73 m².

8. The method according to any one of claims 1 to 7, wherein the patient has an eGFR ≥20 mL/min/1.73 m² and a urinary albumin-to-creatine ratio (UACR) ≥200 mg/g.

9. The method according to any one of claims 1 to 5, wherein the patient has an eGFR ≥45 and <90 ml/min/1.73m² and a urinary albumin-to-creatine ratio (UACR) ≥ 200mg/g.

10. The method according to any one of claims 1 to 9, wherein the patient is treated with a RAAS inhibitor.

11. The method according to any one of claims 1 to 10, wherein the patient is treated with an Angiotensin-Converting Enzyme (ACE) inhibitor and/or an angiotensin II receptor blocker (ARB).

12. The method according to any one of claims 1 to 11, wherein the patient is a non-diabetic patient.

13. The method according to any one of claims 1 to 12, wherein the patient is a non-diabetic and non-pre-diabetic patient.

14. The method according to any one of claims 1 to 13, wherein the patient is a patient with pre-diabetes or a patient with type 2 or type 1 diabetes mellitus.

15. The method according to any one of claims 1 to 14, wherein empagliflozin is administered at a dose in a range from 1 mg to 25 mg.

16. The method according to any one of claims 1 to 15, wherein empagliflozin is administered at a dose of 10 mg or 25 mg.

17. The method according to any one of claims 1 to 16, wherein empagliflozin is administered once daily to the patient.
